(19) Europäisches Patentamt
European Patent Office
Office européen des brevets

(11) Veröffentlichungsnummer: **0 144 830**
**B1**

(12) # EUROPÄISCHE PATENTSCHRIFT

(45) Veröffentlichungstag der Patentschrift: **16.09.87**

(51) Int. Cl.⁴: **C 07 C 97/10**

(21) Anmeldenummer: **84113762.3**

(22) Anmeldetag: **14.11.84**

(54) **Verfahren zur Herstellung von 4,4'-Diaminobenzophenonen.**

(30) Priorität: **24.11.83 DE 3342462**

(43) Veröffentlichungstag der Anmeldung: **19.06.85 Patentblatt 85/25**

(45) Bekanntmachung des Hinweises auf die Patenterteilung: **16.09.87 Patentblatt 87/38**

(84) Benannte Vertragsstaaten: **CH DE FR GB LI**

(56) Entgegenhaltungen:
**AU - B - 484 222**
**DE - A - 3 319 650**
**DE - B - 1 102 176**
**DE - C - 289 108**

(73) Patentinhaber: **BASF Aktiengesellschaft, Carl-Bosch-Strasse 38, D-6700 Ludwigshafen (DE)**

(72) Erfinder: **Disteldorf, Walter, Dr., Portugieser Weg 17, D-6706 Wachenheim (DE)**
Erfinder: **Eisfeld, Wolfgang, Dr., Dubliner Strasse 6, D-6700 Ludwigshafen (DE)**
Erfinder: **Kohler, Rolf-Dieter, Dr., Amselweg 3, D-6803 Edingen-Neckarhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 144 830 B1

**Beschreibung**

Die Erfindung betrifft ein Verfahren zur Herstellung von 4,4'-Diaminobenzophenonen der Formel I

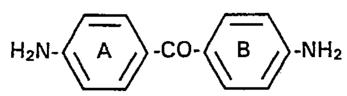

in der die Ringe A und B noch substituiert sein können, das dadurch gekennzeichnet ist, dass man Verbindungen der Formel II

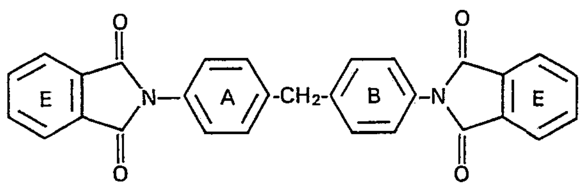

in der die Ringe A, B und E durch unter den Reaktionsbedingungen inerte Substituenten substituiert sein können, in Gegenwart von Nitrobenzol oder einer Alkansäure mit wässriger Salpetersäure unter Überdruck oxidiert und die Reaktionsprodukte spaltet.

Die Ringe A und B können z.B. durch Fluor, Chlor, Brom oder Nitro substituiert sein. Für die Ringe E sind als Substituenten beispielsweise Fluor, Chlor, Brom, Carboxyl oder Nitro zu nennen. Für die Umsetzung geeignete Alkansäuren haben insbesondere 2 bis 4 C-Atome, im einzelnen seien beispielsweise Mono-, Di- oder Trihalogenessigsäure, Buttersäure und vorzugsweise Essigsäure oder Propionsäure genannt.

Unter wässriger Salpetersäure wird erfindungsgemäss eine 0,5 bis 15%ige, vorzugsweise 1 bis 4%ige Salpetersäure verstanden. Zweckmässigerweise wird diese Salpetersäurekonzentration durch Zugabe von konzentrierter Salpetersäure zum Reaktionsgemisch aus Alkansäure, Wasser und Verbindung der Formel II eingestellt.

Die erfindungsgemässe Umsetzung wird zweckmässigerweise bei Temperaturen von 100 bis 200°C, vorzugsweise 150 bis 170°C so vorgenommen, dass der Druck $>1$ bar ist. Nach oben ist der Druck durch die Konstruktion des Reaktionsgefässes bestimmt. Eine Ausbeuteabhängigkeit vom Druck wurde nicht gefunden.

Die Verwendung von Alkansäuren ist gegenüber Nitrobenzol bevorzugt, weil man so ein reineres Produkt erhält.

Die Spaltung der Reaktionsprodukte der Reaktion kann nach bekannten Methoden vorgenommen werden.

Einzelheiten des erfindungsgemässen Verfahrens können den Beispielen entnommen werden, in denen sich Angaben über Teile und Prozente, sofern nicht anders vermerkt, auf das Gewicht beziehen.

Die Verbindungen der Formel I sind wertvolle Zwischenprodukte zur Herstellung von Farbstoffen und Kunststoffen.

Die Verbindungen der Formel II kann man nach an sich bekannten Methoden durch Umsetzung von Verbindungen der Formel

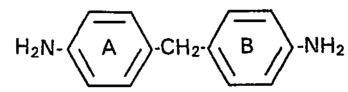

mit Anhydriden der Formel

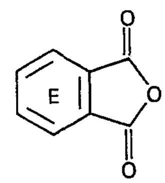

herstellen.

*Beispiele 1 bis 7*

Es werden die in Tabelle 1 aufgeführten Diphenylmethane jeweils mit 200 g Alkansäure in einem salpetersäurebeständigen Rührautoklaven mit Rückflusskühler und Druckkonstanthaltung oberhalb des Kühlers unter Aufpressen von Inertgas auf 160°C erhitzt, wobei die Druckregelung auf 7 bar eingestellt wird. Dann werden unter Temperatur und Druckeinstellung innerhalb von 0,5 Stunden 35,7 g 53%ige Salpetersäure zugepumpt, wodurch sich eine Salpetersäurekonzentration von 3% einstellt. Dann wird noch 1 Stunde unter den gleichen Reaktionsbedingungen nachoxidiert. Die $HNO_3$-Endkonzentration beträgt 0,9%. Der entspannte Oxidationsaustrag wird auf Raumtemperatur abgekühlt, der ausgefallene Feststoff abgesaugt, mit Wasser gewaschen und bei 80 bis 100°C und 20 mbar getrocknet. Man erhält jeweils das entsprechende Benzophenon als 99%ige Ware. Die Ausbeuten sind in Tabelle 1 aufgeführt.

TABELLE 1

| Beispiel Nr. | Diphenylmethan | Menge (g) | Alkansäure | Ausb. (Mol.%) |
|---|---|---|---|---|
| 1 | 4,4'-Bis-phthalimidoyldiphenylmethan | 45,8 | Propionsäure | 78,5 |
| 2 | 4,4'-Bis-(4-carboxiphthalimidoyl)-diphenylmethan | 54,6 | Propionsäure | 91,4 |
| 3 | 4,4'-Bis-(4-chlorphthalimidoyl)-diphenylmethan | 52,7 | Propionsäure | 82,6 |
| 4 | 4,4'-Bis-(3-chlorphthalimidoyl)-diphenylmethan | 52,7 | Propionsäure | 81,5 |

TABELLE 1 (Fortsetzung)

| Beispiel Nr. | Diphenylmethan | Menge (g) | Alkansäure | Ausb. (Mol.%) |
|---|---|---|---|---|
| 5 | 4,4'-Bis-(4,5-dichlorphthalimidoyl)-diphenylmethan | 59,6 | Propionsäure | 86,4 |
| 6 | 4,4'-Bis-phthalimidoyldiphenylmethan | 45,8 | Essigsäure | 71 |
| 7 | 4,4'-Bis-phthalimidoyl-2,2'-bis-chlor-diphenylmethan | 52,7 | Propionsäure | 80,3 |

*Beispiel 8*

54,6 g 4,4'-Bis-(4-carboxiphthalimidoyl)-diphenylmethan werden analog Beispiel 1 in 200 g Essigsäure bei 140°C und 5 bar oxidiert und aufgearbeitet. Man erhält 96%iges 4,4'-Bis-(4-carboxiphthalimidoyl)-benzophenon in 74% Mol.% Ausbeute.

*Beispiel 9*

45,8 g 4,4'-Bis-phthalimidoyl-diphenylmethan werden analog Beispiel 1 in 200 g Nitrobenzol bei 160°C und 7 bar oxidiert. Im Oxidationsaustrag wird das Nitrobenzol durch Wasserdampfdestillation entfernt. Man erhält 85%iges 4,4'-Bis-phthalimidoylbenzophenon in 73 Mol.% Ausbeute.

*Beispiel 10*

23 g 4,4'-Bis-phthalimidoylbenzophenon werden in 46 g Monoethanolamin 1 Stunde bei 80°C gerührt. Nach Abkühlung auf 20°C werden 200 g Wasser zugegeben. Das ausgefallene Produkt wird abgesaugt, mit Wasser gewaschen und im Vakuum getrocknet. Man erhält >99%iges 4,4'-Diaminobenzophenon in 90 Mol.% Ausbeute vom Fp. 241°C.

*Beispiel 11*

27,3 g 4,4'-Bis-(4-carboxiphthalimidoyl)-benzophenon werden analog Beispiel 10 gespalten. Man erhält >99%iges 4,4'-Diaminobenzophenon in 92 Mol-% Ausbeute vom Fp. 241°C.

Entsprechend Beispiel 10 können auch die weiteren Verbindungen der Formel II in die Aminoverbindungen überführt werden.

**Patentansprüche**

1. Verfahren zur Herstellung von 4,4'-Diaminobenzophenonen der Formel I

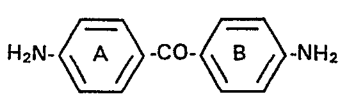

in der die Ringe A und B noch substituiert sein können, dadurch gekennzeichnet, dass man Verbindungen der Formel II

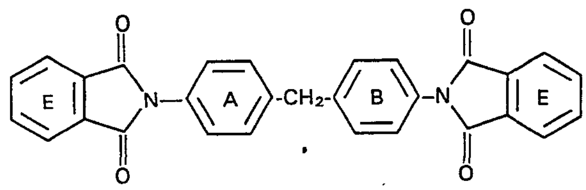

in der die Ringe A, B und E durch unter den Reaktionsbedingungen inerte Substituenten substituiert sein können, in Gegenwart von Nitrobenzol oder einer Alkansäure mit wässriger Salpetersäure unter Überdruck oxidiert und die Reaktionsprodukte spaltet.

2. Verfahren gemäss Anspruch 1, dadurch gekennzeichnet, dass man in Gegenwart von Alkansäuren oxidiert.

3. Verfahren gemäss Anspruch 1 und 2, dadurch gekennzeichnet, dass man in Gegenwart von Essigsäure oder Propionsäure oder Gemischen dieser Säuren oxidiert.

4. Verfahren gemäss Anspruch 1, dadurch gekennzeichnet, dass man die Oxidation bei 0,5 bis 15%iger, vorzugsweise 1 bis 4%iger Salpetersäurekonzentration durchführt.

5. Verfahren gemäss Anspruch 1, dadurch gekennzeichnet, dass man die Oxidation bei Temperaturen zwischen 150 und 170°C durchführt.

**Claims**

1. A process for the preparation of a 4,4'-diaminobenzophenone of the formula I

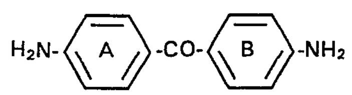

where the rings A und B may be further substituted, wherein a compound of the formula II

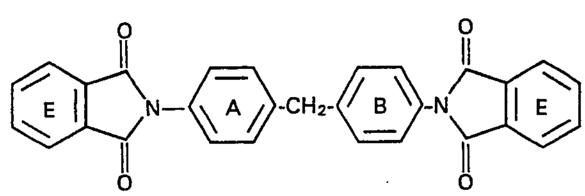

where the rings A, B und E may bear substituents which are inert under the reaction conditions, is oxidized with aqueous nitric acid in the presence of

nitrobenzene or of an alkanoic acid unter superatmospheric pressure, and the reaction product is cleaved.

2. A process as claimed in claim 1, wherein the oxidation is carried out in the presence of an alkanoic acid.

3. A process as claimed in claim 1 and 2, wherein the oxidation is carried out in the presence of acetic or propionic acid or a mixture of these acids.

4. A process as claimed in claim 1, wherein the oxidation is carried out using nitric acid having a concentration of from 0.5 to 15%, preferably from 1 to 4%.

5. A process as claimed in claim 1, wherein the oxidation is carried out at from 150 to 170°C.

## Revendications

1. Procédé de préparation de 4,4'-diaminobenzophénones de formule I

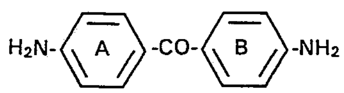

dans laquelle les noyaux A et B peuvent être encore substitués, caractérisé en ce qu'on oxyde des composés de formule II

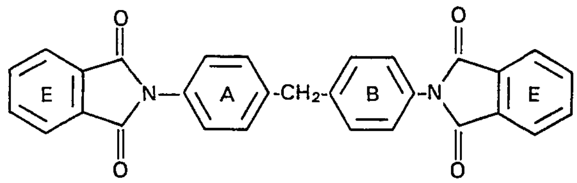

dans laquelle les noyaux A, B et E peuvent être substitués par des substituants inertes dans les conditions de la réaction, avec de l'acide nitrique aqueux sous surpression en présence de nitrobenzène ou d'un acide alcanoïque et on scinde les produits de la réaction.

2. Procédé selon la revendication 1, caractérisé en ce qu'on oxyde en présence d'acides alcanoïques.

3. Procédé selon la revendication 1 ou 2, caractérisé en ce qu'on oxyde en présence d'acide acétique, d'acide propionique ou de mélanges de ces acides.

4. Procédé selon la revendication 1, caractérisé en ce qu'on effectue l'oxydation avec un acide nitrique concentré à 0,5-15%, de préference à 1-4%.

5. Procédé selon la revendication 1, caractérisé en ce qu'on effectue l'oxydation à des températures comprises entre 150 et 170°C.